Europäisches Patentamt

European Patent Office

Office européen des brevets

Publication number: **0 288 833**

**A1**

# EUROPEAN PATENT APPLICATION

(21) Application number: 88105991.9

(22) Date of filing: 14.04.88

(51) Int. Cl.⁴ **A61B 17/22**

(30) Priority: 30.04.87 US 45077

(43) Date of publication of application:
**02.11.88 Bulletin 88/44**

(84) Designated Contracting States:
**CH DE FR GB IT LI NL**

(71) Applicant: **ADVANCED CARDIOVASCULAR SYSTEMS, INC.**
**1395 Charleston Road**
**Mountain View, CA 94039-7101(US)**

(72) Inventor: **The inventors have agreed to waive their entitlement to designation**

(74) Representative: **Baillie, Iain Cameron et al**
**c/o Ladas & Parry Isartorplatz 5**
**D-8000 München 2(DE)**

(54) Balloon dilatation catheter with laser cutting capability.

㊼ Balloon dilatation catheter (11) with laser cutting capabilities having a flexible elongate tubular member (12) with proximal and distal extremities (13, 14). The tubular member has at least one lumen (16) extending therethrough. A fiber optic element (21) extends through the lumen. A metallic tube (22) encloses a substantial portion of the fiber optic element (21) to provide increased rigidity and also to enhance pushability. A coil (26) is wrapped onto the distal extremity of the fiber optic element and a ball-like tip (32) is secured to the distal extremity of the fiber optic element (2). An inflatable balloon (4) is carried by the distal extremity of the tubular member. The tubular member is formed with a flow passage which is in communication with the interior of the balloon for inflating and deflating the balloon.

FIG.—2

## BALLOON DILATATION CATHETER WITH LASER CUTTING CAPABILITY

### Technical Field

This invention relates to balloon dilatation catheters and more particularly to balloon dilatation catheters having laser cutting capabilities.

### Background of the Invention

Balloon dilatation catheters have been heretofore provided. In addition, ball tip catheters have been developed to maximize laser ablation of atheromatous tissue to minimize adjacent thermal injury. There however has not been developed, and there is a need for balloon dilatation catheters which have laser cutting capabilities.

Briefly and in general terms the present invention provides a balloon dilatation catheter with laser cutting capabilities having a flexible elongate tubular membrane with proximal and distal extremities. The tubular member has at least one lumen extending therethrough. A metallic tube is disposed in the lumen and has a hole extending therethrough. An optical fiber extends through the hole in the metallic tube and has a distal extremity which extends beyond the metallic tube. A coil of wire has one end secured to the distal extremity on the metallic tube and is wound around the optical fiber to a region adjacent to distal extremity of the optical fiber. A ball-like tip is secured to the distal extremity of the optical fiber. An inflated balloon is carried by the distal extremity of the tubular member. Means is carried by the tubular member forming a flow passage which is in communication with the interior of the balloon for inflating and deflating the balloon.

### The Drawings

Figure 1 is a side elevational view of a balloon dilatation catheter with laser cutting capabilities incorporated in the present invention.

Figure 2 is an enlarged cross-sectional view of the distal extremity of the catheter shown in Figure 1.

Figure 3 is an enlarged cross-sectional view taken along the line 3-3 of Figure 2.

### Detailed Description

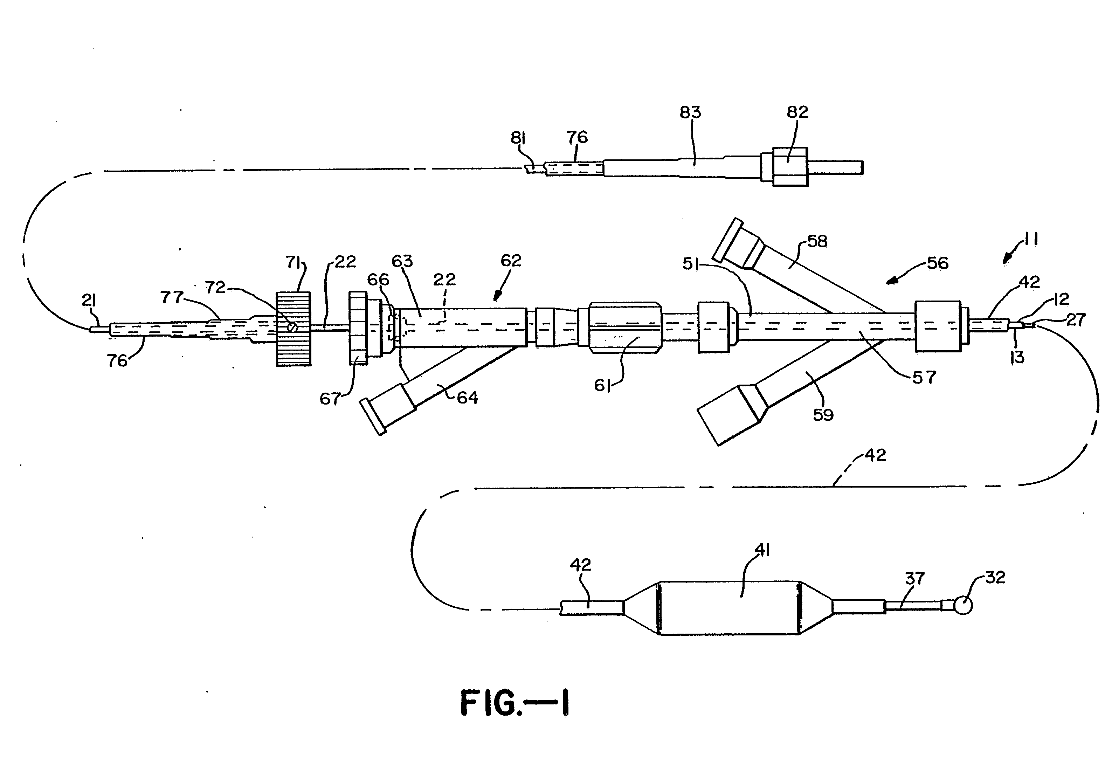

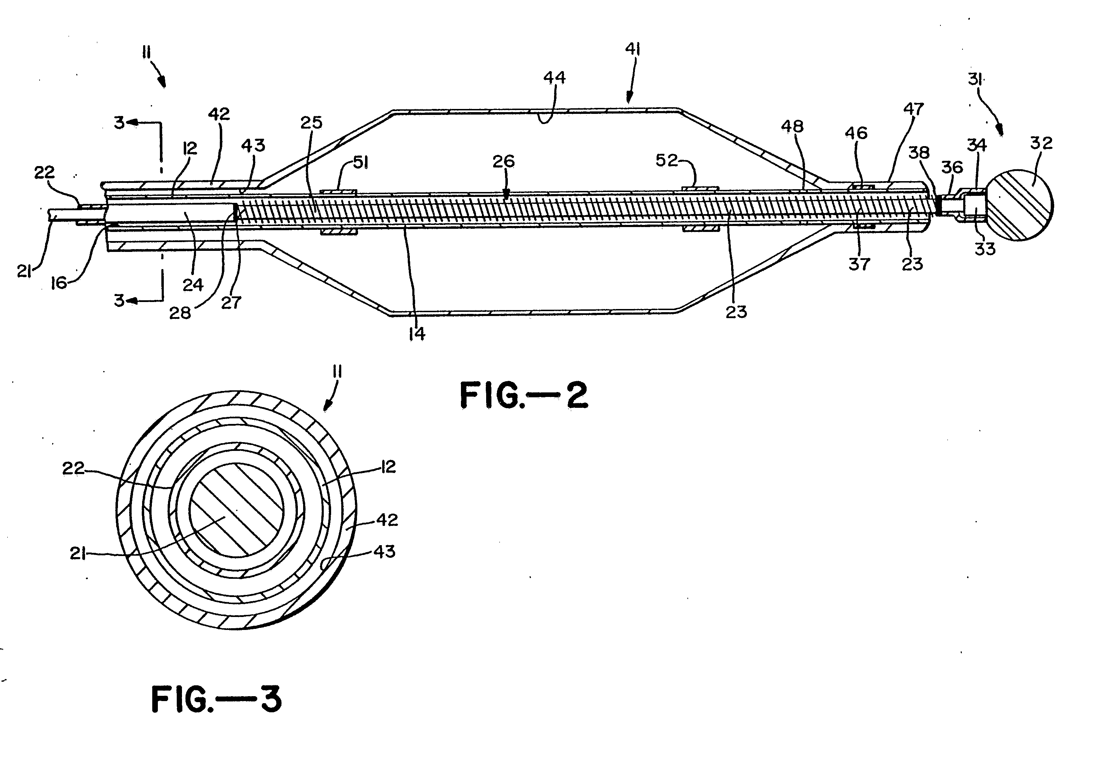

As shown in Figures 1 through 3 of the drawings, the balloon dilatation catheter 11 with laser cutting capabilities comprises an elongate flexible tubular member 12 having proximal and distal extremities 13 and 14. The tubular member 12 can be formed of a suitable material such as polyethylene tubing. The tubular member 12 can be of various sizes depending on the size of the vessel it is desired to negotiate with the catheter. By way of example, the tubular member 12 can have an outside diameter of 1.27 mm (.050 inches) and an inside diameter of 1.02 mm (.040 inches). The tubular member 12 is provided with a lumen 16 extending the length thereof.

A fiber optic element or assembly 21 is provided which is adapted to extend through the lumen 16. The element can have a suitable diameter such as .3 mm (.012 inches) and can have a suitable length as for example a length of 170 to 175 cm. In order to give additional rigidity to the fiber optic element 21 and to enhance its pushability, a substantial portion of the fiber optic element is disposed within a metallic tube 22 formed of a suitable material such as stainless steel. The stainless steel tube which also can be called a hypo tube with a fiber optic element 21 having an outside diameter of .30 mm (.012 inches) can have an inside diameter of .33 mm (.013 inches) and an outside diameter of .76 mm (.030 inches). The hypo tube 22 can have a suitable length as for example 120 cm. The distal extremity 23 of the fiber optic element 21 can extend a suitable distance as for example 10 cm beyond the distal extremity 24 of the hypo tube 22. In order to provide sufficient flexibility for the distal extremity 23 of the fiber optic element 21 and to make it capable to withstand bending. A coil 26 of suitable material such as stainless steel wire of a suitable diameter as for example .076 mm (.003 inches) is wound onto the distal extremity 23 of the fiber optic element 21 with densely packed turns. The proximal extremity 27 of the coil 26 is secured to the distal extremity 24 of the hypo tube 22 by suitable means such as epoxy 28. The densely packed turns of the coil 24 are packed with each turn abutting another turn throughout the length of the coil 26 which extends over the distal extremity 23 of the fiber optic element 21.

A ball-like tip asembly 31 is provided which is secured to the distal extremity 23 of the fiber optic element. The ball-like tip assembly 31 consists of a ball 32 formed of a suitable material such as silica which is bonded to a silica stem 33. A metallic cup-like member 34 formed of a suitable material such as gold extends over the stem 33 and is bonded to a short metallic tube 36 formed of a suitable material such as stainless steel and having an inside diameter of .33 mm (.013 inches) and an outside diameter of .46 mm (.018 inches). The tube 36 extends to the stem and is secured to the distal extremity 37 of the coil 26 and is bonded thereto by suitable means such as an epoxy 38. The stem 33 is secured to the distal extremity 23 of the fiber optic element 21. A suitable coating such as a polyimide coating can be applied to the exterior surface of the tubular sleeve 36 and the metallic cup-like member 34. The ball 32 can have a suitable diameter as for example from 1.2 to 3.0 mm. However it should be appreciated that if desired larger balls can be utilized. The ball, if desired, can have a diameter which is larger than the inside diameter of the tubular member 12 which forms the lumen 16 which makes the fiber optic assembly 21 non-removable.

An inflatable balloon 41 is carried by the distal extremity of the elongate flexible tubular member 12. The balloon 41 as shown can be formed integral with an elongate flexible tubular member 42 which extends coaxially of the elongate flexible tubular member 12. The balloon 41, if desired, can be formed as a separate element and bonded to the tubular member 42. An annular flow passage 43 is formed between the flexible tubular member 12 and the tubular member 42 and is in communication with the interior 44 of the balloon 41 so that the balloon 41 can be inflated and deflated by introducing a balloon inflation medium and withdrawing the same through the annular flow passage 43.

A marker 46 is provided at the distal extremity of the tubular member 12 and consists of a suitable radio opaque material such as a gold band having an inside diameter of .76 mm (.030 inches) and an outside diameter of .91 mm (.036 inches). The marker 46 is mounted on the exterior of the tubular member 12 and is retained thereon by heat shrinking the distal extremity 47 of the balloon 41 onto the distal extremity 14 of the elongate flexible tubular member 12 to provide a liquid tight seal therebetween. Small vent holes 48 of a suitable diameter such as 1 mm can be provided from the interior 44 of the balloon and extending to ambient between the distal extremity 47 of the balloon and the distal extremity 14 of the tubular member 12 in the manner disclosed in the United States Patent No. 4,638,805. These self-venting holes 48 bleed air from the interior 44 of the balloon 41 when it is being filled but prevent the escape of liquid from the balloon 41.

Additional spaced apart markers 51 and 52 are provided for indicating the proximal and distal portions of the balloon 41 and are positioned within the balloon 41 on the tubular member 12 and also can take the form of gold bands or alternatively in the form of platinum wire wrapped onto the tubular member 12.

The proximal extremity 13 of the tubular member 12 as well as the tubular member 42 are secured to a three arm adapter 56 which is provided with a central arm 57 and side arms 58 and 59. The central arm 57 is in communication with the lumen 16 of the tubular member 12. The side arm 58 is in communication with the annular flow passage 43 in the tubular member 42. The other side arm 59 is in communication with the central lumen and can be used for perfusion.

A rotary hemostatic valve 61 is mounted on the central arm 57 and has the fiber optic element 21 with its hypo tube 22 covering the same extending through the same. A two arm adapter 62 is mounted on the rotary hemostatic valve 61 and is provided with a central arm 63 and a side arm 64. The hypo tube 22 with its fiber optic element 21 extends through the central arm 63 and through an O-ring 66 mounted within the central arm. They also extend through a knob 67 which is adapted to engage to O-ring to compress the same to form a liquid tight seal between the O-ring and the hypo tube 22. The side arm 64 is in communication with the central arm 63 and can be utilized for introducing a flushing medium such as a saline solution into the catheter 11.

A torque knob 71 is mounted on the hypo tube 22 and is secured thereto by a set screw 72 and can be utilized for rotating the hypo tube 22 and the fiber optic element 21 carried therein. The fiber optic element 21 extends beyond the torque knob 71 and is encased in an elongate flexible tubular member 76 formed of a suitable material such as polyethylene having a suitable diameter such as an inside diameter of .05 mm (.0020 inch) and an outside diameter of .1 mm (.0040 inch). Also, it can have a suitable length as for example a length of approximately 50 cm. A length of heat shrinkable tubing 77 can be provided on the tubular member 76 for making the transition from the torque knob 71 and to provide additional reinforcing. The proximal extremity 81 of the fiber optic element 21 is secured to a suitable connector as for example a conventional male SMA connector 82. A length of heat shrinkable tubing 83 is provided for establishing reinforcing at the point at which the tubular member 76 is connected to the connector 82.

Operation and use of the balloon dilatation catheter 11 with laser cutting capabilities may now be briefly described as follows. Prior to insertion of the catheter 11 into the body of the patient, the balloon 41 should be inflated to insure that all air has been removed therefrom. This can be accomplished by inserting the balloon inflation medium through the side arm 58 and introducing it into the interior 44 of the balloon 41 to cause any air therein to be expelled through the vent holes 48. The holes 48 as explained previously are of a size so that they permit the escape of air but prevent the escape of liquid from the interior 44 of the balloon 41. A guiding catheter (not shown) is then inserted into the vessel of the patient to be utilized for guiding the catheter 11. Thereafter if the ball is smaller than the catheter lumen, with the fiber optic assembly 19 removed from the catheter 11, a guide wire of a conventional type can be inserted into the central lumen 57 in the three arm adapter and extended through the lumen 16. The guide wire then can be utilized in a conventional manner to advance the catheter 11 beyond the guiding catheter. The guide wire can be advanced into the stenosis and then the catheter 11 can be advanced so that the balloon 41 is within the stenosis. A suitable balloon inflation medium can then be introduced into the side arm 58 to inflate the balloon 41.

After the balloon 41 has been positioned in the stenosis, the balloon 41 can be inflated by introducing a balloon inflation medium through the side arm 58 to inflate the balloon for a suitable time at the desired pressure. After the balloon inflation has been utilized to create a larger opening in the stenosis, the balloon 41 can be deflated and withdrawn.

If it is desired to perform additional operations on the stenosis by laser cutting, the guide wire can be removed and the fiber optic assembly 19 inserted through the lumen 16 until the ball-like member 32 extends beyond the distal extremity of the catheter 11. The ball-like tip 32 is self guiding and greatly reduces possible perforation of the side wall of the vessel being negotiated. The ball-like tip 32 facilitates negotiation of tortuous vessels after the ball-like tip 32 has been appropriately positioned.

If the ball-like tip 32 is larger than the catheter lumen 16, a separate guidewire cannot be used. The fiber optic assembly can then be assembled into the catheter 11 as shown in Figure 2 so that it is a part of the catheter 11. The fiber optic assembly 13 with its ball-like member 32 can act as a guide wire for guiding the catheter 12 through tortuous vessels.

Laser energy can then be introduced by a laser (not shown) connected to the SMA connector 82 causing an appropriate amount of energy to be directed down the fiber optic assembly 19 and into the silica ball tip 32 to cause ablation of the atheromatous tissue in the stenosis while at the same time minimizing adjacent thermal injury to the vessel. During this procedure, the tissue being irradiated can be immersed in a suitable saline solution by introducing the saline solution into the side arm 64 of the two arm adapter 62.

It should be appreciated that various types of procedures can be accomplished with the catheter of the present invention which has laser cutting capabilities in performing in vivo laser angioplasty. If a vessel is totally occluded, the silica ball tip can be utilized for ablating the tissue forming the stenosis to form at least a small opening therethrough. Thereafter if desired, the balloon 41 can be advanced into the opening formed in the stenosis and further enlarged by inflating the balloon 41 for a desired time at an appropriate pressure. The ball-like tip 32 in addition to making it possible to deliver substantial amounts of energy to the area in which ablation is to occur is also efficacious in that is minimizes adjacent thermal injury. In addition the ball-like tip 32 because of its rounded configuration inhibits perforation of the vessel upon which the procedure is being performed.

It is apparent from the foregoing that there has been provided a balloon-type dilatation catheter which has laser cutting capabilities which can be utilized in in vivo angioplasty. The ball-like tip is self-guiding which facilitates its movement in tortuous vessels while eliminating the possibility of mechanical perforation of the vessel. The fiber optic element has sufficient pushability but retains sufficient flexibility so the tortuous vessels can be negotiated.

## Claims

1. In the balloon dilatation catheter with laser cutting capabilities, a flexible elongate tubular member having proximal and distal extremities, the tubular member having a least one lumen extending therethrough, a fiber optic element extending through the lumen, a metallic tube enclosing a substantial portion of the fiber optic element to provide increased rigidity and also to enhance pushability, a coil wrapped on the distal extremity of the fiber optic element and a ball-like tip secured to the distal extremity of the fiber optic element, an inflatable balloon carried by the distal extremity of the tubular member, and means carried by the tubular member forming a flow passage in communication with the interior of the balloon for inflating and deflating the balloon.

2. A catheter as in Claim 1 wherein said means carried by the tubular member forming a flow passage in communication with the interior of the balloon includes an additional tubular member extending coaxially over the first named tubular member to provide an annular flow passage therein which is in communication with the interior of the balloon.

3. A catheter as in Claim 1 wherein said balloon is formed integral with the additional tubular member.

4. A catheter as in Claim 1 together with a coil of wire wrapped about the distal extremity of the fiber optic element.

5. A catheter as in Claim 4 wherein one end of the coil is secured to the distal extremity of the metallic tube and the other end is secured adjacent to the ball-like tip carried by the fiber optic element.

6. A catheter as in Claim 1 together with a torque knob secured to the proximal extremity of the metallic tube together with an additional flexible tube extending proximally of the torque knob and wherein said fiber optic element extends into the additional flexible tube.

7. A catheter as in Claim 1 together with an adapter secured to the proximal extremity of the elongate tubular member, the adapter having a central arm and a side arm, the fiber optic element extending through the central arm and wherein the flow passage for inflating the balloon is coupled to the side arms.

8. A catheter as in Claim 7 together with a rotating hemostatic valve mounted on the central arm of the adapter, an additional adapter secured to the rotating hemostatic valve, and having a central arm and a side arm, wherein the metallic tube extends through the central arm of the additional adaptor and wherein the torquing device is secured to the metallic tube.

9. A catheter as in Claim 1 wherein the ball-like tip is formed of silica.

10. A catheter as in Claim 9 wherein the ball-like tip includes a ball and a stem secured to the ball and wherein the fiber optical element is in communication with the stem.

11. A catheter as in Claim 11 together with a metallic cup enclosing the stem.

12. A catheter as in Claim 1 wherein the ball-like tip has a diameter which is greater than the diameter of the lumen.

FIG.—1

0 288 833

FIG.—2

FIG.—3

0 288 833

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 217 165 (FOX et al.)<br>* Page 1, lines 17-23; page 7, lines 17-27; page 9, lines 19-23 *<br>--- | 1 | A 61 B 17/22 |
| A | US-A-4 418 688 (LOEB)<br>* Column 2, lines 44-54; figures 1,2 *<br>--- | 1 | |
| A | EP-A-0 136 365 (STORZ-ENDOSKOP)<br>* Page 5, lines 2-4 *<br>--- | 1 | |
| A | EP-A-0 164 623 (SUMITOMO ELECTRIC)<br>* Page 3, lines 19-24 *<br>--- | 1 | |
| A,D | US-A-4 638 805 (POWELL)<br>----- | 1 | |

TECHNICAL FIELDS SEARCHED (Int. Cl.4)

A 61 M
A 61 B

The present search report has been drawn up for all claims

| Place of search | Date of completion of the search | Examiner |
|---|---|---|
| THE HAGUE | 04-08-1988 | RAKOWICZ,J.M. |

EPO FORM 1503 03.82 (P0401)